Europäisches Patentamt

European Patent Office

Office européen des brevets

(11) Numéro de publication: **0 226 506**
**A2**

(12) ## DEMANDE DE BREVET EUROPEEN

(21) Numéro de dépôt: **86402658.8**

(22) Date de dépôt: **01.12.86**

(51) Int. Cl.⁴: **A 61 K 35/78**
**A 61 K 37/22**

(30) Priorité: **06.12.85 FR 8518084**

(43) Date de publication de la demande:
**24.06.87 Bulletin 87/26**

(84) Etats contractants désignés:
**AT BE CH DE ES GB GR IT LI LU NL SE**

(71) Demandeur: **Desjonqueres, Stéphane**
**7 rue du Chant des Oiseaux**
**F-78360 Montesson (Les Yvelines) (FR)**

(72) Inventeur: **Desjonqueres, Stéphane**
**7 rue du Chant des Oiseaux**
**F-78360 Montesson (Les Yvelines) (FR)**

(74) Mandataire: **Cabinet Pierre HERRBURGER**
**115, Boulevard Haussmann**
**F-75008 Paris (FR)**

(54) **Composition médicamenteuse destinée à être utilisée dans les indications de la rhumatologie et de la traumatologie.**

(57)    a) Composition médicamenteuse destinée à être utilisée dans les indications de la rhumatologie et de la traumatologie :

b) caractérisée en ce qu'elle contient au moins une huile naturelle d'origine venégtale hyperoxy-génée par saturation en oxygène et exposition intensive et contrôlée aux ultra-violets de façon à présenter un taux de péroxydation compris entre 30 et 300 exprimé en milliéquivalents et une teneur en glycérides oxydés comprise entre 5 et 40. la définition de cette huile en spectrophotométrie UV étant de 1 à 6 pour le facteur D 270 et de 8 à 60 pour le facteur E 232.

EP 0 226 506 A2

**Description**

" Composition médicamenteuse destinée à être utilisée dans les indications de la rhumatologie et de la traumatologie "

La présente invention se rapporte à une composition médicamenteuse destinée à être utilisée dans les indications de la rhumatologie et de la traumatologie.

La possibilité d'utiliser des péroxydes de corps gras pour le traitement de certaines affections a été mentionnée pour la première fois dans le brevet français n° 2 330 M : l'utilisation des compositions décrites dans ce document n'a malheureusement pas permis d'aboutir aux résultats espérés, notamment à cause de problèmes de tolérance, et pour cette raison, les recherches visant à améliorer ces compositions ont été interrompues pendant un certain nombre d'années.

Ces recherches ont été reprises récemment, en liaison avec l'intérêt accru du public pour les produits naturels, et en particulier, pour la médecine dite "douce".

On a, ainsi, pu mettre en évidence des qualités pharmacologiques des huiles hyperoxygénées, pouvant déboucher sur un certain nombre d'applications. Le brevet français n° 2 461 744 décrit, par exemple, un procédé de péroxydation de matières grasses pouvant aboutir à la préparation de compositions utilisables dans le domaine médical.

Conformément à l'invention, on s'est aperçu, de manière surprenante, que certains corps gras péroxydés appartenant à la famille susmentionnée, possédaient des qualités propres leur permettant des se montrer nettement supérieurs aux médicaments analogues utilisés jusqu'à présent dans les indications de la rhumatologie et de la traumatologie qu'il s'agisse d'allopathie ou d'autres produits d'origine naturelle.

A cet effect, l'invention concerne une composition médicamenteuse renfermant de tels corps gras.

Cette composition est caractérisée en ce qu'elle contient au moins une huile naturelle d'origine végétale hyperoxygénée par saturation en oxygène et exposition intensive et contrôlée aux ultraviolets de façon à présenter un taux de péroxydation compris entre 30 et 300 exprimé en milli-équivalents et une teneur en glycérides oxydés comprise entre 5 et 40, la définition de cette huile en spectrophotométrie UV étant de 1 à 6 pour le facteur E 270 et de 8 à 60 pour le facteur E 232. (NF T 60 223).

Les qualités particulières de cette composition, qui renferme uniquement des produits naturels, sont basées sur une triple activité anti-inflammatoire, antalgique et cicatrisante.

L'activité anti-inflammatoire a été mise en évidence à partir d'un protocole expérimental traditionnel d'irritation locale de l'oreille du rat avec l'huile de croton ; au cours de ces expériences, l'activité de la composition objet de l'invention a été comparée avec celle de l'indométacine qui est l'anti-inflammatoire utilisé classiquement ; cette étude réalisée chez le rat a permis de conclure que l'administration topique de lacomposition objet de l'invention était capable de limiter de façon nette la phlogose induite localement par un agent irritant.

L'activité antalgique de cette composition a pu être démontrée par massage de témoins avec des huiles hyperoxygénées conformes à l'invention dans le cadre d'études cliniques en double aveugle, où le produit de comparaison était un anesthésiant de contact clairement défini par la pharmacopée française.

Des études cliniques réalisées en double aveugle contre placebo et d'autres travaux réalisés en ouvert, ont permis de mettre en lumière une activité cicatrisante indéniable, commune à toutes les huiles d'origine végétale hyperoxygénées répondant à la définition ci-dessus.

Selon une autre caractéristique de l'invention, la ou les huils(s) hyperoxygénée(s) est (sont) choisies dans le groupe formé par l'huile d'arachide, l'huile d'amande douce et l'huile de carthame.

Il a été trouvé que ces huiles présentent à un dégré élevé, la triple activité anti-inflammatoire, antalgique et cicatrisante susmentionnée, et, en même temps se distinguent pa une excellente tolérance et une absence totale de toxicité.

En effet, tous les travaux effectués dans ce sens (détermination de l'indice d'irritation primaire cutanée chez le lapin, détermiantion de l'indice d'irritation occulaire, étude de la toxicité transmuqueuse chez le lapin, recherche d'éventuels pouvoirs allergènes...) ont amené sans exception à conclure par un constat d'absence totale d'irritation cutanée ou de tout autre tolérance allergique ou non, qu'il s'agisse de la peau ou des muqueuses.

En pharmacologie humaine, tous les travaux cliniques entrepris ont confirmé sans exception les facteurs de tolérance mis en évidence par les travaux réalisés sur l'animal.

Parmi les huiles hyperoxygénées mentionnées ci-dessus, on a trouvé que l'huile d'arachide hyperoxygénée possédait des propriétés antalgiques et anti-inflammatoires en faisant un produit particulièrement actif dans le traitement des infections suivantes : tendinite, rachialgie, algie arthrosique, algie inflammatoire, arthrite subaigüe, algie musculaire et péri-articulaire, rhumatisme ab-articulare, traumatologie, choc, contusion, hématome, conditions postopératoires ...

Cette activité remarquable est due à la présence dans l'huile d'arachide, d'acide arachidonique, acide gras dont la structure est semblable aux médiateurs lipidiques de l'inflammation ; il est possible que cet acide agisse par compétition au niveau des sites récepteurs de ces médiateurs, diminuant ainsi l'inflammation locale.

Par ailleurs, l'oxygène présent dans la composition et libéré dans le derme, qui est constitué uniquement d'oxygène non toxique (c'est-à-dire dépourvu de radicaux libes) pourraît agir au niveau des terminaisons nerveuses dermiques en empêchant le processus douloureux de se former, celui-ci semblant résulter de l'anoxie au niveau des récepteurs épidermiques de la douleur.

Bien entendu, la composition médicamenteuse objet de l'invention peut n'être constituée que par une ou plusieurs huiles naturelles d'origine végétale hyperoxygénées répondant à la définition mentionnée ci-dessus ; cependant, et selon une autre caractéristique de l'invention, cette composition se présente aussi sous la forme de gel.

A titre d'exemple, un gel conforme à l'invention peut comporter entre 90 et 95 % d'huile d'arachide hyperoxygénée entre 1 et 2 % de parfum et entre 4 et 8 % d'un excipient à base d'huile d'amande douce.

Un gel s'étant montré particulièrement satisfaisant répond à la composition suivante :

Hule d'arachide hyperoxygénée .. 92,5 %
Parfum ......................... 1,5 %
Aerosil 300 (marque déposée) .... 6,0 %

L'Aérosil 300 est un excipient composé de silice commercialisé par la Société DEGUSSA FRANCE.

L'emploi de l'électrothérapie se combine parfaitement avec l'utilisation de la composition conforme à l'invention.

Il est remarquable de constater que les huiles hyperoxygénées contenues dans la composition objet de l'invention sont dénuées de présence de radicaux libres cytotoxiques qui sont généralement associés à tout corps oxydé ; cette absence est de nature à justifier l'emploi de ces compositions en médecine humaine ; en effect, l'agressivité des radiaux libres sur la peau est bien connue et leur contibution à l'apparitation de cancer a été fréquemment mentionnée.

Il est, en outre, essentiel de mentionner que les compositions objet de l'invention ne sont pas limitées à la présence des huiles susmentionnées, et que toutes les huiles végétales péroxydées répondant à la défiition ci-dessus peuvent parfaitement être utilisées dans le cadre des compositions conformes à l'invention.

L'activité des compositions conformes à l'invention sera mise en lumière grâce à l'exemple ci-dessous qui est un compte-redu d'essai en double aveugle d'une huile d'arachide péroxydée dans les états algo-inflammatoires de l'appareil locomoteur, par comparaison à un placebo.

Cet essai a eu pour but d'étudier l'activité et la tolérance de l'huile d'arachide péroxydée comparativement à un placebo dans diverses manifestations algiques et/ou inflammatories de l'appareil locomoteur.

Cet essai a été réalisé dans un service de rééducation fonctionnelle, chez 50 sujets, à savoir : 21 subjets de sexe masculin et 29 subjets de sexe féminin, dont l'âge était compris entre 17 et 76 ans avec une moyenne de 48 ans et la répartition suivante :

- moins de 20 ans : 3 cas
- de 20 à 40 ans : 15 cas
- de 40 à 60 ans : 21 cas
- plus de 60 ans : 1 cas.

Les 50 sujets traitées souffraient de divers manifestations algiques et/ou inflammatoires de l'appareil locomoteur, se répartissant comme suit :
- Algies musculaires et péri-articulaires..20 cas
- Algies traumatiques, post-fracuraires ou post-opératoires.........................19 cas
- Algies arthrosiques......................7 cas
- Algies inflammatories....................3 cas
- Névralgies sciatiques....................1 cas.

L'essai a été conduit en double aveugle sur deux séries de malades auxquels a été attribué, après tirage au sort,, soit la composition objet de l'invention (25 cas) soit un placebo de présentation identique (25 cas).

Le produit a été uniquement utilisé en administration locale sous forme d'application par massage deux fois par jour ; la durée du traitement a varié de 48 heures à plus de 8 jours en fonction du résultat obtenu.

Il n'a jamais été prescrit parallèlement ni antalgique ni anti-inflammatoire.

L'efficacité de la thérapeutique a été jugée sur l'évolution de la symptomatologie algo-inflammatoire estimée à l'aide des critères cliniques suivants :
- douleur.
- chaleur,
- tuméfaction,
- raideur,
- mobilité.

Les résultants obtenus sont résumés dans le tableau suivant :

0 226 506

| RESULTATS | COMPOSITION CONFORME A L'INVENTION | | PLACEBO | |
|---|---|---|---|---|
| Excellents (+++) | 11 | 44 % | 4 | 16 % |
| Bons (++) | 13 | 52 % | 8 | 32 % |
| Légers (+) | 0 | | 10 | 40 % |
| Nuls (0) | 1 | 4 % | 2 | 8 % |
| Aggravation | 0 | | 1 | 4 % |
| | 25 | | 25 | |

Le tableau ci-dessus montre que le pourcentage de résultats satisfaisants (c'est-à-dire excellents et bons) a été nettement supérieur pour ce qui concerne la composition objet de l'invention (96 %) par rapport au placebo (48 %).

L'invention propose donc une composition médicamenteuse parfaitement tolérée, exempte de toute toxicité et qui permet l'automédication.

Par comparaison, les produits d'allopathie par massage qui se prévalent d'un effet anti-inflammatoire : cortisoniques et anti-inflammatoires non stéroidiens (A.I.N.S.) sont tous soumis à la législtation sur les produits toxiques dont la délivrance au malade exige une prescription médicale.

**Revendications**

1°) Composition médicamenteuse destinée à être utilisée dans les indications de la rhumatologie et de la traumatologie, caractérisée en ce qu'elle contient au moins une huile naturelle d'origine végétale hyperoxygénée par saturation en oxygène et exposition intensive et contrôlée aux ultra-violets de façon à présenter un taux de péroxydation compris entre 30 et 300 exprimé en milli-équivalents et une teneur en glycérides oxydés comprise entre 5 et 40, la définition de cette huile en spectrophotométrie UV étant de 1 à 6 pour le facteur E 270 et de 8 à 60 pour le facteur E 232.

2°) Composition médicamenteuse selon la revendication 1, caractérisée en ce que la ou les huile(s) hyperoxygénée(s) est(sont) choisie(s) dans le groupe formé par l'huile d'arachide, l'huile d'amande douce et l'huile de carthame.

3°) Composition médicamenteuse selon l'une quelconque des revendications 1 et 2, caractérisée en ce que l'huile hyperoxygénée est l'huile d'arachide.

4°) Composition médicamenteuse sous forme de gel selon l'une quelconque des revendications 1 à 3, caractérisée en ce qu'elle contient de l'huile hyperoxygénée, le reste étant constitué par un excipient composé de silice.

5°) Composition médicatmenteuse selon l'une quelconque des revendications 1 à 4, caractérisée en ce qu'elle sse présente sous la forme d'un gel.

6°) Composition médicamenteuse selon la revendication 5, se présentant sous la forme d'un gel, caractérisée en ce qu'elle contient entre 90 et 95 % d'huile d'arachide hyperoxydénée, entre 1 et 2 % de parfum et entre 4 et 8 % d'un excipient à base d'huile d'amande douce.

4